# EUROPEAN PATENT SPECIFICATION

(11) **EP 1 214 085 B1**
(45) Date of publication and mention of the grant of the patent: **26.01.2005**
(21) Application number: 00965967.3
(22) Date of filing: 08.09.2000
(51) Int. Cl.: A61K 35/78, A61P 9/00

(54) **AN ANTIOXIDANT PREPARATION BASED ON PLANT EXTRACTS FOR THE TREATMENT OF CIRCULATION AND CHRONIC DEGENERATIVE PROBLEMS AND OF HYPERTENSION**
EIN ANTIOXYDIERUNGSMITTEL AUF BASIS VON PFLANZENEXTRAKTEN ZUR BEHANDLUNG VON KREISLAUFS- UND CHRONISCHEN DEGENERATIVEN PROBLEMEN UND VON BLUTHOCHDRUCK
PREPARATION D'ANTIOXYDANT A BASE D'EXTRAITS VEGETAUX DESTINEE AU TRAITEMENT DES PROBLEMES LIES A LA CIRCULATION ET AU PROCESSUS DEGENERATIF CHRONIQUE, ET AU TRAITEMENT DE L'HYPERTENSION

(30) Priority: 10.09.1999 CH 166499
(43) Date of publication of application: 19.06.2002
(73) Proprietor: Ceteris Holding B.V.-Amsterdam (Olanda) - Succursale di Lugano, 6900 Lugano (CH)
(72) Inventor: MERIZZI, Gianfranco, I-10128 Torino (IT)
(74) Representative: Rambelli, Paolo
(86) International application number: PCT/EP2000/008876
(87) International publication number: WO 2001/019381

(56) References cited:
- GB-A- 2 174 904
- W.REILLY, V.REEVE: "Body contouring using an oral herbal antioxidant formulation-Centelaplus: a dose controlled observational study" REDOX REPORT, vol. 5, no. 2-3, 2000, pages 144-145, XP000990069

## Description

The present invention relates to a preparation based on plant extracts which has an antioxidant effect and is particularly useful in the prevention and treatment of circulation and chronic-degenerative problems, and in the prevention and treatment of hypertension.

The object of the invention is to provide a preparation to be taken orally, based on a combination of active ingredients of natural and plant origin which, when administered orally work more effectively to prevent and treat the aforesaid problems.

This object is achieved according to the invention by providing a preparation characterised in that its active ingredients include a combination of *Ginkgo biloba* biflavones, catechine and/or epicatechine, cumarine and derivatives thereof, and an ingredient chosen from asiaticoside, asiatic acid, madecassic acid and compounds thereof.

The preparation is obtained by mixing plant extracts which contain the above active principles.

It is known that extracts from the leaves of *Ginkgo biloba* contain important active principles and in particular flavonol glucosides, lactonic terpenes and dimeric biflavones or flavones. The flavonol glucosides and the lactonic terpenes constitute the active components of standardized *Ginkgo biloba* extracts currently available on the market and are, respectively, powerful antioxidants and stimulants of nitric oxide and of effective platelet aggregating factor (PAF) antagonists. Thanks to the combined action of the active principles they contain, standard *Ginkgo biloba* extracts have proved to have a powerful vaso-motor effect, able to improve both central and peripheral blood flow. However, these extracts do not contain the biflavone component which is not extracted during normal processing. The Ginkgo biloba extract used in preparations according to the present invention is highly enriched with the biflavone component and, as a possible option, with extracts containing flavonol glucosides and lactonic terpenes. Five biflavones in particular have been identified in the biflavone component of *Ginkgo biloba:* these are, in particular, amentoflavone, bilobetine, isoginkgetine, ginkgetine and sciadopisitine; the five said compounds differ only by the presence of methyl compounds in some positions and, like all flavones, are powerful antioxidants. However, from a pharmacological point of view, they are characterised by their anti-phosphodiesterase, anti-inflammatory, vasculokinetic and anti-allergy properties. Phosphodiesterases (PDE) are cell enzymes responsible for interacting with cyclic nucleotides so as to linearize them. Cyclic nucleotides are involved as second messengers in transmitting intercellular signals and are thus responsible for some phenomena which are very important from a biochemical point of view. They assist with the visual process and in the relaxation of smooth muscles, they stimulate lipolysis in adiposity and vasculo-motion in capillary arterioles. More specifically, it is sufficient to report that in inhibiting PDE depending on cyclic AMP, these biflavones demonstrate an IC50 of 1.2 micromoles.

The anti-inflammatory properties of biflavones, and in particular those of amenthoflavone, have been demonstrated both in vitro, by measuring the interaction of these biflavones with cyclo-oxygenase, lipo-oxygenase e phospholipase A2, and in vivo, using various models of inflammation in animals (carragineen oedema, Croton oil inflammation etc). The anti-inflammatory action of biflavones was confirmed both in models using local application and in those in which they were administered intraperitoneally. In these models, the biflavones always demonstrated an anti-inflammatory action equivalent to that of indomethacyn or prednisolone. This effectiveness can be explained by analizing the IC50 of cyclo-oxygenase inhibition, which is 3 micromoles for amentoflavone.

With regard to the microvascularkinetic activity of biflavones, it should be reported that, following acute treatment, these substances improve the size of the arterial sphygma wave and, following chronic treatment they improve capillary density in tissues with trophic-connective problems, such as those affected by panniculopathy and/or various degrees of sclerodermy. Biflavones also have clear anti-allergy properties; they inhibit the release of histamine by mast-cells stimulated by allergens: thereby reducing or countering the formation of oedemas resulting from vasodilation and increases in vascular permeability.

In the context of the present invention, it has been demonstrated that, when administered orally, the activity of the aforesaid biflavones, possibly in combination with flavonol glucosides and lactonic terpenes which are normally present in standard *Ginkgo biloba* extracts, is enhanced when the latter are combined with the aforesaid active principles.

The extracts are preferably used in a phytosomal form, in which the active components are compounded with phospholipids.

In the context of the invention it is convenient to use an extract of leucocyanidine or leucoanthocyanin derived from *Vitis vinifera* as the source of catechine or epicatechine. Leucoanthocyanins are procyanidolic oligomers derived from condensing monomeric units of flavan-3-ols and flavan-3,4-diols, these being either free or esterified with gallic acid; leucoanthocyanines are powerful antioxidants. They are able to protect the endothelial wall of vessels and the extra-cellular matrix surrounding capillary walls, as well as having anti-atherosclerotic properties owing to their antioxidant action on low-density lipoproteins (LDL) in blood.

These active principles have a good bio-availability even when administered orally and their tropism have been demonstrated for the cardio-vascular system and for all tissues, such as artery walls, which are rich in glycoamminoglycene.

Preferably, phytosomal forms of extracts are used, thus further enhancing the bioavailability of the active principles. In this form the procyanidines are complexed with phospholipids, particularly with soya distearoylphosphatidyecholine.

The preferable source of cumarine is an extract of *Melilotus* officinalis, cumarine and its derivatives being the main active principles thereof; the main active principles of this extract are melilotine (3.4 dihydro-cumarine), melilotic acid (hydroxycumarinic acid), melilotoside (melilotin glucoside) and some flavonoids which act like vitamin P; the active ingredients contained in the extract are particularly effective in increasing capillary strength, in reducing vascular permeability, in stimulating venous circulation and improving lymphatic circulation.

Extract of Melilotus may be replaced or backed up, as a source of cumarine and its derivatives, by an extract of *Aesculus hippocastanum* (horse chestnut) in the same dosage or up to around twice the dose of Melilotus extract.

The most abundant active ingredient of *Aesculus hippocastanum* extract, obtained from the bark, the pericarp of the fruit, the leaves or the buds, is cumarine glucoside, esculoside (6-0-glucosil-7-hydroxy-cumarine).

Other cumarines contained in the extract are fraxine (8-0-glycoside-7-hydroxy-6-mehoxycumarine) and aglicone, esculetine (6,7-dioxy-cumarine) and fraxetine (7,8-dioxy-6-methoxy-cumarine).

The preferred source of asiaticoside, asiatic acid and madecassic acid is an extract containing a triterpene fraction of centella (*Centella asiatica*) which contains a combination of the above three active principles. The extract should preferably be used in a phytosomal form, obtained by a reaction between the triterpene fraction of the *Centella asiatica* with a phospholipid. A main action of the triterpene fraction of centella consists in accelerating the uptake and metabolism of lysine and of proline, thus increasing the synthesis and the release of tropocollagen and stimulating the turnover of acid mucopolysaccharides in connective tissue.

The basic composition of the invention can thus be obtained by mixing a Ginkgo biloba biflavone extract (perhaps in combination with a standard *Ginkgo biloba* extract also containing flavonol glucosides and lactonic terpenes), leucocyanidine extract, *Melilotus officinalis* extract and Centella extract; these extracts preferably being in a phytosomal form except for the *Melilotus officinalis* extract.

With reference to the extracts normally available on the market, the basic composition is preferably made up by the following percentages by weight:
2.5 - 40% *Ginkgo biloba* biflavone extract;
15 - 80% of leucocyanidine extract;
2.5 - 60%, preferably 2.5 - 30% of *Melilotus officinalis* and/or *Aesculus hippocastanum* extract;
2.5 - 40% of centella extract; possibly in combination with:
2.5 - 40% of standard *Ginkgo biloba* extract containing flavonol glucosides and lactonic terpenes.
In terms of the content of active principles, the composition of the invention preferably contains the following percentages by weight:
0.2 - 14%, preferably 0.8 - 5% of total biflavones, expressed as ginkgetine content,
0.5- 16%, preferably 1.5 - 6% of catechine and/or epicatechine, expressed as catechine content;
0.1 - 6%, preferably 0.4 - 2% of cumarine and its derivatives;
0.3 - 18%, preferably 0.9 - 6% of asiaticoside;
0.4 - 26%, preferably 1.4 - 9 % of asiatic acid and/or madecassic acid;
and possibly one or more of the following substances:
0.2 - 10%, preferably 0.6 - 4%, of flavonol glucosides and
up to 1.3- 2%, preferably up to 0.5%, of ginkgolide lactonic terpenes (bilobalide).

The composition can also contain active ingredients chosen from gamma-linolenic acid, eicosapentaenoic acid (EPA), docohexaenoic acid (DHA), ruscogenin and/or neoruscogenin, flavinoids such as vitexine, hyoside, proanthocyanidine, epicatechine and crategolic acid and mixtures thereof.

Gamma-linolenic acid is preferably introduced into the preparation in borage oil, added in quantities of 50 to 180% by weight with reference to 100 parts of basic mixture.

The preferred source of eicosapentaenoic acid (EPA) and of docohexaenoic acid (DHA) is fish oil which, with reference to 100 parts of the basic composition, may be added in quantities of 25 to 120% by weight.

The preferred source of ruscogenin and/or neoruscogenin is an extract of *Ruscus aculeatus* (Butcher's broom), this extract is preferably added in quantities of 5 to 50% by weight, with reference to 100 parts of the basic mixture.

The preferred source of flavonoids is an oily maceration of hawthorn *Crataegus oxyacantha* which, with reference to 100 parts of the basic mixture, can be added in quantities from 25 to 100% by weight.

In particular, in the preferred embodiment of the invention, the composition includes one or more of the following components in the following percentage amounts referred to the total composition:
3 - 36%, preferably 10-12% of gamma-linolenic acid;
2 - 36%, prererably 7 - 12% of eicosapentaenoic acid;
1.5 - 24%, preferably 5 - 8 % of docohexaenoic acid;
0.1 - 6%, preferably 0.4 - 2% of ruscogenin and/or neoruscogenin; and
up to 0.4%, preferably up 0.2% of flavonoids, expressed as a quantity of hyoside.

For example, a typical composition could be formulated according to the data in the table below, which gives the preferred minimum and maximum quantities by weight of the components of the basic mixture (marked-with an asterisk) and of optional ingredients.

| | **Minimum** (Parts by weight) | **Maximum** (Parts by weight) |
|---|---|---|
| *Dry extract of *Vitis vinifera* (optionally phytosomes) | 20 | 200 |
| Oily maceration of hawthorn | 20 | 100 |
| *Dry extract of *Centella asiatica* (optionally phytosomes) | 20 | 100 |
| ***Dry extract of *Melilotus officinalis* and/or *Aesculus hippocastanum* | 5 | 40 |
| Dry extract of *Ruscus aculeatus* | 5 | 100 |
| Dry extract of *Ginkgo biloba* (optionally phytosomes) | 10 | 75 |
| *Dimeric flavones of *Ginkgo biloba* (optionally phytosomes) | 10 | 75 |
| Borage oil | 50 | 1000 |
| Fish oil | 50 | 750 |
| Soya lecithin | 20 | 1000 |
| Dosage 1-3 capsules per day. | | |

In the above table, the given values, expressed in parts by weight, correspond, when expressed in milligrams to the minimum and maximum recommended daily doses or to the dose per capsule.

The preparation of the invention is formulated in forms suited to be taken orally, such as, for example, gelatin capsules with either soft or hard cases, tablets, pills, elixirs, suspensions and syrups. The mix of extracts can be administered orally, possibly in an edible vehicle or can be incorporated directly into food as part of a diet.

The composition is particularly useful in the prevention and treatment of circulation and chronic degenerative problems caused by damage to the vascular endothelium, the extracellular matrix or to surrounding tissues of the arterial, venous or lymphatic systems.

In the arterial system, such damage can be translated, for example, into reactions causing the formation of atherotomes leading to atherosclerosis, to the onset of ischemic processes due to the a narrowing of the arteries and to the onset of thrombotic problems caused by an atherome possibly becoming detached. In the venous system, dilation and loss of permeability of the vessels can, for example, cause chronic venous insufficiency and the onset of venous thrombotic troubles. In addition, some problems affecting the venous system can be a result of damage to lymphatic vessels, which, among other things, are responsible for draining tissues and circulating lymph.

The compositon of the invention provides an association of substances which are well understood from both a pharmacological and a clinical point of view, which is totally free of side effects and is particularly well suited to the treatment and the prevention of the main problems affecting the circulation system, including the heart, and that of chronic degenerative problems linked thereto.

Clinical trials have also shown that the preparation is able to reduce both arterial and diastolic blood pressure and is thus particularly useful in the treatment and prevention of hypertension.

## Claims

1. A composition based on plant extracts, with an antioxidant activity which is particularly useful in the prevention and treatment of circulation and chronic degenerative problems and in the prevention and treatment of hypertension, **characterised in that** its active ingredients comprise, in association, biflavones of *Ginkgo biloba,* catechine and/or epicatechine, cumarine and/or derivatives thereof and a component chosen from among madecassic acid, asiatic acid, asiaticoside or combinations thereof.

2. A composition according to Claim 1, **characterised in that** it is obtained by mixing plant extracts containing the aforesaid active principles.

3. A composition according to Claim 2, characterised that the said extracts are in phytosomal form.

4. A composition according to any Claim from 1 to 3, **characterised in that** it also includes flavonol glucosides and lactonic terpenes.

5. A composition according to any Claim from 1 to 4, **characterised in that** it also includes an active principle chosen from a group consisting of gamma-linolenic acid, icosapentaenoic acid, docohexaenoic acid, ruscogenin and/or neoruscogenin, flavonoids and combinations thereof.

6. A composition according to Claim 5, in which the said flavonoids are selected from among vitexine, hyoside, proanthocyanidine, epicatechine, crategolic acid and combinations thereof.

7. A composition according to any one of Claims 1 to 4, **characterised in that** it is obtained by mixing plant extracts in the following percentages by weight:
2.5-40% of *Ginkgo biloba* biflavone extract;
15-80% of leucocyanidine extract;
2.5-30% of Melilotus and/or *Aesculus byppocastanum* extract;
2.5-40% of centella extract; and optionally
2.5-40% of standardised *Ginkgo biloba* extract containing flavone glucosides and lactonicoterpenes.

8. A composition according to Claim 7, **characterised in that** with reference to 100 parts by weight of the basic mixture of Claim 7, it also includes one or more of the following components:
from 50 to 180% by weight of borage oil;
from 25 to 120% by weight of fish oil;
from 5 to 50% by weight of *Ruscus aculeatus* (Butcher's broom) extract; and
from 25 to 100 % by weight of a maceration of *Crataegus oxyacantha* (hawthorn).

9. A composition according to any one of the preceding Claims which includes:
0.2-14%, preferably 0.8-5% by weight, of total biflavones;
0.5-16%, preferably 1.5-6% by weight, of catechine and/or epicatechine;
0.1-6%, preferably 0.4-2% by weight, of cumarine and derivatives thereof;
0.3-18%, preferably 0.9-6% by weight of asiaticoside;
0.4-26%, preferably 1.4-9% by weight, of asiatic acid and/or madecassic acid; and optionally
0.2-10%, preferably 0.6-4% by weight, of flavonol glucosides and
up to 1.3%, preferably up to 0.5% by weight, of lactonic terpenes.

10. A composition according to Claim 9, **characterised in that** it also includes one or more of the following components:
3 - 36% wt, preferably 10 - 12% of gamma-linolenic acid;
2 - 36% wt, preferably 17 - 12% of eicosapentanoic acid;
1.5 to 24% wt, preferably 5 - 8% of docohexaenoic acid;
0.1- 6% wt preferably 0.4 - 2% of ruscogenin and/or neoruscogenin; and
up to 0.4% wt, preferably up to 0.2% of flavonoids.

11. A composition according to any one of the preceding Claims in a pharmaceutical form for oral administration.

## Patentansprüche

1. Zusammensetzung auf Basis von Pflanzenextrakten mit oxidationshemmender Wirkung, welche besonders nützlich ist bei der Verhinderung und Behandlung von Kreislaufproblemen und chronischen degenerativen Problemen und bei der Verhinderung und Behandlung von Bluthochdruck, **dadurch gekennzeichnet, dass** ihre Wirkstoffe, in Verbindung miteinander, Biflavone von *Ginkgo biloba,* Catechin und/oder Epicatechin, Cumarin und/oder Derivate davon und einen Bestandteil, der ausgewählt ist aus Madecassic-Säure, Asiatic-Säure, Asiaticosid oder Kombinationen davon, umfassen.

2. Zusammensetzung gemäß Anspruch 1, **dadurch gekennzeichnet, dass** sie durch Vermischen von Pflanzenextrakten, welche die obengenannten aktiven Grundbestandteile enthalten, erhalten wird.

3. Zusammensetzung gemäß Anspruch 2, **dadurch gekennzeichnet, dass** die Extrakte in phytosomaler Form vorliegen.

4. Zusammensetzung gemäß einem Anspruch von 1 bis 3, **dadurch gekennzeichnet, dass** sie auch Flavonolglucoside und Lactonterpene enthält.

5. Zusammensetzung gemäß einem Anspruch von 1 bis 4, **dadurch gekennzeichnet, dass** sie auch einen aktiven Grundbestandteil enthält, der ausgewählt ist aus einer Gruppe, bestehend aus Gamma-Linolensäure, Eicosapentaensäure, Docosahexaensäure, Ruscogenin und/oder Neoruscogenin, Flavonoiden und Kombinationen davon.

6. Zusammensetzung gemäß Anspruch 5, wobei die Flavonoide ausgewählt sind aus Vitexin, Hyperosid, Proanthocyanidin, Epicatechin, Crategolsäure und Kombinationen davon.

7. Zusammensetzung gemäß einem der Ansprüche 1 bis 4, **dadurch gekennzeichnet, dass** sie durch Vermischen von Pflanzenextrakten in den folgenden Gewichtsanteilen erhalten wird:
2,5 - 40 % *Ginkgo biloba*-Biflavon-Extrakt;
15 - 80 % Leucocyanidin-Extrakt;
2,5 - 30 % Melilotus- und/oder *Aesculus hyppocastanum-*Extrakt;
2,5 - 40 % Centella-Extrakt; und gegebenenfalls
2,5 - 40 % standardisierter *Ginkgo biloba*-Extrakt, enthaltend Flavonglucoside und Lactonterpene.

8. Zusammensetzung gemäß Anspruch 7, **dadurch gekennzeichnet, dass** sie, bezogen auf 100 Gewichtsteile der Grundmischung nach Anspruch 7, auch einen oder mehrere der folgenden Bestandteile enthält:
50 - 180 Gew.% Borretschöl;
25 -120 Gew.% Fischöl;
5 - 50 Gew.% *Ruscus aculeatus* (Stechender Mäusedorn)-Extrakt; und
25 -100 Gew.% eines Mazerats von *Crataegus oxyacantha* (Weißdorn).

9. Zusammensetzung gemäß einem der vorhergehenden Ansprüche, welche Folgendes umfasst:
0,2 - 14, vorzugsweise 0,8 - 5, Gew.% Gesamtbiflavone;
0,5 - 16, vorzugsweise 1,5 - 6, Gew.% Catechin und/oder Epicatechin;
0,1- 6, vorzugsweise 0,4 - 2, Gew.% Cumarin und Derivate davon;
0,3 -18, vorzugsweise 0,9 - 6, Gew.% Asiaticosid;
0,4 - 26, vorzugsweise 1,4 - 9, Gew.% Asiatic-Säure und/oder Madecassic-Säure; und gegebenenfalls
0,2 - 10, vorzugsweise 0,6 - 4, Gew.% Flavonolglucoside und
bis zu 1,3, vorzugsweise bis zu 0,5, Gew.% Lactonterpene.

10. Zusammensetzung gemäß Anspruch 9, **dadurch gekennzeichnet, dass** sie auch einen oder mehrere der folgenden Bestandteile enthält:
3 - 36, vorzugsweise 10 - 12, Gew.% Gamma-Linolensäure;
2 - 36, vorzugsweise 17 - 12, Gew.% Eicosapentansäure;
1,5 - 24, vorzugsweise 5 - 8, Gew.% Docosahexaensäure;
0,1 - 6, vorzugsweise 0,4 - 2, Gew.% Ruscogenin und/oder Neoruscogenin; und
bis zu 0,4, vorzugsweise bis zu 0,2, Gew.% Flavonoide.

11. Zusammensetzung gemäß einem der vorhergehenden Ansprüche in pharmazeutischer Form zur oralen Verabreichung.

## Revendications

1. Composition à base d'extraits végétaux ayant une activité anti-oxydante, qui est particulièrement utile dans la prévention et le traitement de problèmes de circulation et de dégénérescence chronique et dans la prévention et le traitement de l'hypertension, **caractérisée en ce que** ses principes actifs comprennent, sous forme associée, des biflavones de *Ginkgo biloba,* de la catéchine et/ou de l'épicatéchine, de la coumarine et/ou des dérivés de. celle-ci et un composant choisi parmi l'acide madécassique, l'acide asiatique, l'asiaticoside ou des combinaisons de ceux-ci.

2. Composition selon la revendication 1, **caractérisée en ce qu'**elle est obtenue en mélangeant des extraits végétaux contenant les principes actifs susmentionnés.

3. Composition selon la revendication 2, **caractérisée en ce que** lesdits extraits sont sous forme de phytosomes.

4. Composition selon l'une quelconque des revendications 1 à 3, **caractérisée en ce qu'**elle comprend également des flavonol-glucosides et des terpènes lactoniques.

5. Composition selon l'une quelconque des revendications 1 à 4, **caractérisée en ce qu'**elle comprend également un principe actif choisi dans le groupe formé par l'acide gamma-linolénique, l'acide icosapentanoique, l'acide docohexanoïque, la ruscogénine et/ou la néoruscogénine, les flavonoïdes et leurs combinaisons.

6. Composition selon la revendication 5, dans laquelle lesdits flavonoïdes sont choisis parmi la vitexine, l'hyoside, la proanthocyanidine, l'épicatéchine, l'acide cratégolique et leurs combinaisons.

7. Composition selon l'une quelconque des revendications 1 à 4, **caractérisée en ce qu'**elle est obtenue en mélangeant des extraits végétaux dans les pourcentages en poids suivants :
2,5% à 40% en poids d'extrait de biflavone de *Ginkgo biloba* ;
15% à 80% en poids d'extrait de leucocyanidine ;
2,5% à 30% en poids d'extrait de *Melilotus* et/ou d'*Aesculus hyppocastanum ;*
2,5% à 40% en poids d'extrait de centelle ; et éventuellement
2,4% à 40% d'extrait de *Ginkgo biloba* normalisé contenant des flavone-glucosides et des terpènes lactoniques.

8. Composition selon la revendication 7, **caractérisée en ce que** par rapport à 100 parties en poids du mélange de base de la revendication 7, elle comprend également un ou plusieurs des composants suivants :
de 50% à 180% en poids d'huile de bourrache ;
de 25% à 120% en poids d'huile de poisson ;
de 5% à 50% en poids d'extrait de *Ruscus aculeatus* (petit houx) ; et
de 25% à 100% en poids d'une macération de Crataegus *oxyacantha* (aubépine).

9. Composition selon l'une quelconque des revendications précédentes qui comprend :
0,2% à 14%, de préférence 0,8% à 5% en poids de biflavones totales ;
0,5% à 16%, de préférence 1,5% à 6% en poids de catéchine ou d'épicatéchine ;
0,1% à 6%, de préférence 0,4% à 2% en poids de coumarine ou ses dérivés ;
0,3% à 18%, de préférence 0,9% à 6% en poids d'asiaticoside ;
0,4% à 26%, de préférence 1,4% à 9% en poids d'acide asiatique et/ou d'acide madécassique ; et éventuellement
0,2% à 10% de préférence 0,6% à 4% en poids de flavonol-glucosides et
jusqu'à 1,3% en poids, de préférence jusqu'à 0,5% en poids de terpènes lactoniques.

10. Composition selon la revendication 9, **caractérisée en ce qu'**elle comprend également un ou plusieurs des composants suivants :
3% à 36% en poids, de préférence 10% à 12% en poids d'acide gamma-linolénique ;
2% à 36% en poids, de préférence 17% à 12% en poids d'acide eicosapentanoique ;
1,5% à 24% en poids, de préférence 5% à 8% en poids d'acide docohexanoique ;
0,1% à 6% en poids, de préférence 0,4% à 2% en poids de ruscogénine et/ou de néoruscogénine ; et
jusqu'à 0,4% en poids, de préférence jusqu'à 0,2% en poids de flavonoïdes.

11. Composition selon l'une quelconque des revendications précédentes dans une forme pharmaceutique pour une administration par voie orale.
